(19) Europäisches Patentamt
European Patent Office
Office européen des brevets

(11) **EP 4 610 924 A1**

(12) **EUROPEAN PATENT APPLICATION**
published in accordance with Art. 153(4) EPC

(43) Date of publication:
**03.09.2025 Bulletin 2025/36**

(21) Application number: **23854486.0**

(22) Date of filing: **17.08.2023**

(51) International Patent Classification (IPC):
*G06T 7/00* (2017.01)   *G06T 7/11* (2017.01)
*G06V 10/764* (2022.01)   *G06V 10/82* (2022.01)
*G06N 3/0464* (2023.01)

(52) Cooperative Patent Classification (CPC):
**A61B 3/14; G06N 3/0464; G06N 3/08; G06T 7/00;
G06T 7/0012; G06T 7/11; G06V 10/764;
G06V 10/82;** G06T 2207/10048; G06T 2207/20081;
G06T 2207/20084; G06T 2207/30041

(86) International application number:
**PCT/CN2023/113527**

(87) International publication number:
**WO 2024/037587 (22.02.2024 Gazette 2024/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.08.2022 CN 202210989506**

(71) Applicants:
• **Shanghai Institute Of Endocrine And Metabolic
Diseases
Shanghai 200020 (CN)**
• **Shangai Baiyi Healthcare Technology Co, Ltd
Shanghai 201203 (CN)**

(72) Inventors:
• **WANG, Weiqing**
**Shanghai 200020 (CN)**
• **NING, Guang**
**Shanghai 200020 (CN)**
• **SHEN, Liyun**
**Shanghai 200020 (CN)**
• **TIAN, Chaonan**
**Shanghai 201203 (CN)**
• **DU, Dong**
**Shanghai 201203 (CN)**

(74) Representative: **Elzaburu S.L.P.
Paseo de la Castellana 259C
Torre de Cristal, planta 28
28046 Madrid (ES)**

(54) **PALPEBRAL FISSURE HEIGHT MEASUREMENT METHOD AND APPARATUS, AND STORAGE MEDIUM**

(57) The application relates to a method, apparatus and storage medium for measuring palpebral fissure height. The method comprises acquiring the first eye position image in front of eyeballs by photography when a user looking straight ahead in a near-infrared light field of 700-1200nm; and segmenting the background, the iris, the sclera and the pupil from the first eye position image by means of neural network training. Extract pupil center from the segmented pupil, and obtain a pupil center line in a vertical direction. The distance between the junction point of the sclera, the iris or the pupil and the background on the pupil center line, and calculating an palperbral fissure height by using the distance.

Fig. 1

**Description**

**CROSS-REFERENCE TO RELATED APPLICATIONS**

This application is a continuation of International Pat. Appl. No. PCT/CN2023/113527, filed on August 17, 2023, which claims priority to Chinese Pat. Appl. No. 202210989506.4, filed on August 18, 2022, the contents of each of which are incorporated by reference herein in their entireties.

TECHNICAL FIELD

The invention relates to the technical field of eye measurement, in particular to palpebral fissure height measurement method, and appratus and storage medium.

BACKGROUND DISCUSSION

Palpebral fissure height (also known as eyelid crack width) refers to the distance between the upper and lower eyelids, passing through the pupil. Clinically, eyelid retraction, including upper and lower eyelid retraction, is a common clinical symptom of thyroid-associated ophthalmopathy (TAO) and an important indicator in a clinical diagnosis. Eyelid retraction may not only cause unacceptable facial damage, but also lead to vision-threatening exposed keratopathy, such as corneal ulcers. Therefore, the measurement of eyelid retraction is very important for clinical diagnosis, and the degree of eyelid retraction can be reflected by measuring the palpebral fissure height.

At present, palpebral fissure height is usually measured on a millimeter scale in the clinic, but errors in the measurement data are easy to make due to inaccurate readings and the influence of the operator's level and habits. However, in the diagnosis and evaluation of TAO patients, a fluctuation of 2 mm often predicts a change in the patient's condition, so measurement accuracy is often required, and the above measurement method can cause missed diagnosis and misdiagnosis of the patient, delaying progress in the treatment of the patient's condition.

In addition, the prior art also uses automatic measurement to determine whether the eyelid retraction occurs or not. For example, in a Chinese patent entitled "Method and Device for Identification of Ocular Signs in Thyroid Related Eye Diseases" (Application No. 202010803761.6, Publication date: October 30, 2020), the cornea and the sclera are identified through image recognition and neural network training, and then the image of the cornea and the sclera determines whether the upper eyelid and the upper margin of the cornea region are exposed to the sclera region, so as to determine whether there is an eyelid retraction. However, this method can cause misjudgments for people with Sanpaku eyes, or those who show the whites of the eyes due to the ocular prominence caused by myopia. For example, in a Chinese patent entitled "Blink frequency analysis method and system based on image processing" (Application No. 201910939612.X, Publication date: February 04, 2020), the iris contour and the sclera contour are determined by taking human eye images, the palpebral fissure boundary is determined by iris contour and sclera contour, and the palpebral fissure height is calculated by the difference between the coordinates of the upper boundary and the lower node point of the palpebral fissure. However, the palpebral fissure height is defined as the distance between the upper and lower eyelids through the center line of the pupil, and the palpebral fissure height determined in the above way may be an oblique distance of the palpebral fissure.

Although there are prior arts that use neural network model training methods to segment eye images, their training models are relatively old, and the training process is time-consuming and occupies large amounts of memory space. Therefore, it is necessary to further improve the technologies.

SUMMARY

In order to overcome the lack of accuracy of measuring the palpebral fissure height in the prior art, a method for measuring the palpebral fissure height is disclosed in this application.

In order to achieve the above purposes, the following technical solutions are adopted in this application:

On one hand, a method for measuring the palpebral fissure height is disclosed, which comprises:

Acquiring a first eye position image in front of eyeballs by photography when a user looking straight ahead in a near-infrared light field of 700-1200 nm;

Segmenting a background, an iris, a sclera and a pupil from the first eye position image by a training method of a neural network;

Extracting a center of the pupil from the segmented pupil, and obtaining a center line of the pupil in a vertical direction;

and

Determine the distance between junction points of the sclera, the iris or the pupil on the central line of the pupil and the background, and calculating the palpebral fissure height using the distance.

Further, the pupil center is an average value of X and Y coordinates of all pupil pixels of segmented from the first eye position image, and the center line of the pupil may be obtained by drawing a vertical line through an average value of the X coordinates.

Further, the training method of the neural network segments the background, the iris, the sclera and the pupil from the first eye position image by adopting a combination of UNet and DenseNet neural network models as the neural network model, taking the first eye position image as an input to the model for the neural network, adopting the UNet neural network model to reduce and increase a dimension of the input, and transmitting an output of a dimensionality reducing block to a corresponding dimensionality increasing block by a skip connection, and extracting each dimensionality reducing block and upsampling each dimensionality increasing block using the DenseNet neural network model.

Further, the method may also include verifying a training effect of the neural network using a loss function, wherein the loss function is a compound loss that comprises focal loss, a generalized dice loss, a surface loss and a boundary aware loss.

The loss function may be calculated as follows:

Compound loss = $a_1 \times$ surface loss + $a_2 \times$ focal loss +$a_3 \times$ generalized dice loss + $a_4 \times$ boundary aware loss $\times$ focal loss

wherein $a_1$, $a_2$, $a_3$ and $a_4$ are hyperparameters, $a_1$ is related to a number of duration(s) during training, $a_2 = 1$, $a_3 = 1 - a_1$, and $a_4 = 20$.

Further, the palpebral fissure height may be calculated as: palpebral fissure height (B) = pixel distance (A) of the palpebral fissure height $\times$ single pixel width and length $\times$distance (D) from the palpebral fissure to a lens of the camera $\div$distance (C) from film in the camera to the lens of the camera, where the pixel distance (A) of the palpebral fissure height is the distance between the intersection of the sclera, the iris, or pupil on the pupil center line and the background.

Specifically, the distance (D) from the palpebral fissure to the lens of the camera may equal the distance from a canthal latch point to the lens of the camera 1, minus an ocular prominence ( or exophthalmia degree) 2.

Further, the ocular prominence ( or exophthalmia degree) 2 may be an average value of a normal exophthalmia degree.

On the one hand, the present application provides a apparatus for measuring palpebral fissure height, including:

An image acquisition module, configured to acquire a first eye position image in front of eyeballs by photography when a user looks straight ahead in a near-infrared light field of 700-1200 nm;

An image segmentation module, configured to adopt a training mode of a neural network to segment the background, the iris, the sclera and the pupil from the first eye position image;

A feature extraction module, configured to extract pupil center from the segmented pupil and obtain a pupil center line in the vertical direction; and

A computing module, configured to calculate the distance between the intersection (or junction point) of the sclera, the iris or the pupil and the background on the pupil center line, and calculate the palpebral fissure height by using the distance.

On another hand, the present application discloses a computer-readable storage medium, configured to have at least one program code that is loaded and executed by a processor to perform the present method of measuring palpebral fissure height.

Compared with the prior art, the proposed technical solution has at least the following beneficial effects:

1. Taking eye images in the near-infrared light field can effectively distinguish the iris, the pupil, the sclera, the background and the caruncula lacrimalis, so as to provide more intuitive and more recognizable images for subsequent neural network training.

2. This application adopts a combination form of UNet and DenseNet neural network models for neural network training, which not only takes advantage of the simple and stable structure of the UNet neural network model, which is widely used in medical image processing, but also applies the DenseNet neural network model in each dimensionality reducing block and dimensionality increasing block of the UNet neural network model. In each dimensionality reducing block and dimensionality increasing block, feature reuse is carried out through a skip connection to improve the feature extraction effect. Moreover, the DenseNet neural network model adopted in this application simplifies the existing DenseNet neural network model, avoiding the problem of slow calculation caused by the traditional DenseNet neural network model, which may be associated with all the previous layers in each layer.

3. The Iris, the pupil, the sclera, the background and the caruncula lacrimalis can be accurately identified through the neural network model, so as to identify the intersection of the sclera, the iris or the pupil on the center of the pupil and the background. The distance between the two junction points is the pixel distance of the palpebral fissure height, and the distance of the palpebral fissure height. can be obtained through geometric calculation.

BRIEF DESCRIPTION OF THE DRAWINGS

Fig. 1 shows a flow chart of a method of measuring palpebral fissure height;

Fig. 2 shows a structure of the adopted neural network model;

Fig. 3 shows a processing flow diagram of a dimensionality reducing block;

Fig. 4 shows the processing flow diagram of a dimensionality increasing block;

Fig. 5 shows a diagram of the principle(s) for calculating the palpebral fissure height; and

Fig. 6 is a schematic diagram of a apparatus for measuring palpebral fissure height.

EXAMPLES

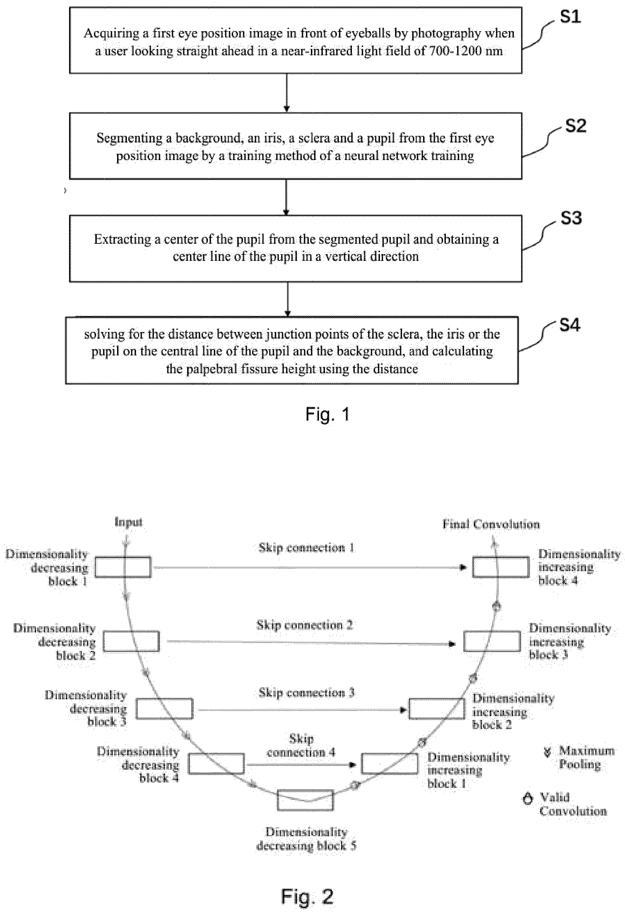

Combined with the attached drawings and examples, the following specific implementation of this application is further described in detail. The following embodiments are used to illustrate, but not to limit, the scope of the application.
Based on the idea of neural network model training, this application extracts the features of the eye image taken to find out the pupil center and further obtain the pupil center line. The pixel distance of the palpebral fissure height is determined according to the intersection of the pupil center line and the eye socket, and the palpebral fissure height is further calculated through imaging principle(s). The concept of this application is further explained by specific embodiments below.
On the one hand, the method of measuring palpebral fissure height as shown in Fig. 1 includes the following steps:

Step S1: Acquiring the first eye position image in front of eyeballs by photography when a user looking straight ahead in a near-infrared light field of 700-1200 nm.
In the above Step S1, the eye image is taken in the near-infrared light field of 700-1200 nm. In the normal visible light band of 400-700 nm, the color of different parts of the eye, namely the pupil, the iris, and the sclera, has almost no effect on the imaging, due to the gradual structure of the limbus of the cornea in the contact part of the iris and the sclera, resulting in an inability to accurately identify the center of the eyeball. The absorption peak of human melanin pigment occurs at about 335 nm, and wavelengths over 700 nm are almost completely unabsorbed, but the reflectivity of the iris is quite stable in near-infrared wavelengths over 700 nm. Therefore, the use of near-infrared light field can easily distinguish boundaries between the sclera, the iris and the pupil. Thus, the accuracy and stability of the method can be improved.

Step S2: Using the training method of neural network to segment the background, the iris, the sclera and the pupil from the first eye position image.

In the above Step S2, the neural network model adopts the combination of UNet and DenseNet neural network models. The neural network model takes the first eye position image as an input, adopts the UNet neural network model to reduce and increase the dimension(s) of the input, and transmits the output of the dimensionality-reducing block(s) to the corresponding dimension increase block(s) by a skip connection. The DenseNet neural network model is used for feature extraction in each dimensionality reducing block, and for upsampling each dimensionality increasing block.

The neural network architecture adopted in this application is a combination of UNet and DenseNet neural network models, which is implemented based on a Pytorch framework. The overall structure of the neural network is a U-shape, as shown in Fig. 2, with a total of five dimensionality reducing blocks, from dimensionality reducing block 1 to dimensionality reducing block 5, and four dimensionality increasing blocks, from dimensionality increasing block 1 to dimensionality increasing block 4.

The neural network receives an input image and passes it to the dimensionality reducing block 1 through 5. With the exception of the dimensionality reducing block 1, each dimensionality reducing block is connected by a maximum pooling layer that reduces the dimension by half. The dimensionality reducing block 5 then passes the output to the dimensionality increasing blocks 1 through 4. The dimensionality increasing block 4 passes the output to a final convolution. Skip connections are listed as follows: the dimensionality reducing block 1 is connected to the dimensionality increasing block 4; the dimensionality reducing block 2 is connected to the dimensionality increasing block 3; the dimensionality reducing block 3 is connected to the dimensionality increasing block 2; and the dimensionality reducing block 4 is connected to the dimensionality increasing block 1.

UNet provides a deep convolutional structure, firstly reducing the input dimension to a certain degree, and then bumping the input back to the original size. At the same time, UNet can transmit information from the dimensionality reducing blocks to corresponding dimensionality increasing blocks by skip connections, and the output of the early layer(s) should be passed to the later layer(s) as an input, which can prevent information loss inside the neural network, and allow maximum learning. On the other hand, DenseNet is more of a concept than a fixed structure. The focus of DenseNet in this application is the specific skip connection technology. An optimized and scaled-down version is proposed in this application. Instead of taking the output of all previous layers as input, as is the case for each layer of the traditional DenseNet network model, each increasing and reducing block performs the skip connection operation 1 or 2 times, which prevents the network from being too slow.

In order to explain the training process of neural networks, the following concepts are introduced:

Max Pooling: Max pooling is a pooling operation that calculates the maximum value in each patch of each feature map. The result is a downsampled or pooled feature map that highlights the most features in the patch, rather than the average presence of features in the average pooled case. This operation corresponds to the torch.nn.MaxPool2d function. The kernel size is defined as $2 \times 2$, and this operation halves the input dimension.

Convolution: Convolution is the simple application of a filter (also known as a kernel) to an input, resulting in produces an activation. Repeated application of the same filter to the input produces an activation graph, called a feature graph, indicating the location and intensity of the detected features in the input. This operation corresponds to the torch.nn.Conv2d function.

Same Convolution: The same convolution is a type of convolution where the output matrix has the same dimension as the input matrix. This operation corresponds to the torch.nn.Conv2d function, which, in the example of this application, has a padding of $1 \times 1$ and a kernel size of $3 \times 3$.

Valid Convolution: A valid convolution is a convolution operation that does not use any padding on the input. This operation corresponds to the torch.nn.Conv2d function, which has no padding in the example of this application and has a kernel size of $1 \times 1$.

Transposed Convolution: The transposed convolution layer attempts to reconstruct the spatial dimension of the convolution layer and inverts the downsampling and upsampling techniques applied to it. This operation corresponds to torch.nn.ConvTranspose2d.

Batch normalization: Batch normalization is a way to make artificial neural networks faster and more stable by re-centering and re-scaling the inputs of layers. This operation corresponds to BatchNorm2d with num_features of 5, which is the number of labels for the network.

Leaky RELU: The leaky correction linear activation function, or simply Leaky RELU, is a linear function that will output an input directly if the input is positive; otherwise, it will output the product of a small factor (0.1 in the example of this application) and the input.

The working process of the specific dimensionality increasing blocks and dimensionality reducing blocks is as follows:

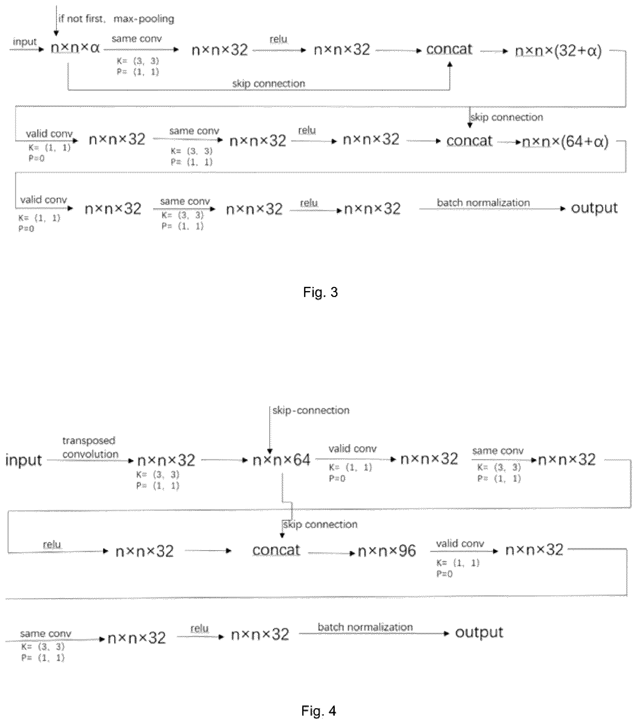

Several convolution operations, as well as Leaky RELU and skip connection operations, are applied to the dimensionality reducing blocks. This application takes the size of the original image as $n \times n \times 1$ dimensions as an example, in which dimension 1 means that the original image has only gray value. A specific structure is shown in Fig. 3.

First, the original input of the dimensionality reducing blocks passes through an identical convolutional Layer (layer-1) with 32 filters (channels). The output of Layer-1 is then subjected to Leaky RELU activation (RELU-Layer-1). Second, RELU-Layer-1 is concatenated with the original input along the channel dimension (Skip-Connection-Input-1), which makes the output dimension 32 plus the dimension of the original input, which is the first case where the skip connection is applied: the original input is passed directly to the later convolution stage.

Third, Skip-Connection-Input-1 is passed to a valid convolution layer (layer-2) with 32 filters, reducing the dimension to 32, which is often referred to as the "bottleneck layer," which shrinks the channel dimension of the neural network to ensure that the network does not become too slow. Fourth, the output of Layer-2 passes again through an identical convolutional layer (Layer-3) with 32 filters (channels). The output of Layer-3 is then subjected to a leaky RELU activation (RELU-Layer-3).

Fifth, RELU-Layer-3 is connected to RELU-Layer-1 and the original input (Skip-Connection-Input-2), which is the case when the skip connection is applied for the second time. Sixth, Skip-Connection-Input-2 again passes through a valid convolution layer (layer-4) with 32 filters, which again reduces the dimension to 32 dimensions. Seventh, the output of Layer-4 is again passed through the same convolutional layer (Layer-5) with 32 filters (channels). A leaky RELU activation (RELU-Layer-5) is then applied to the output of Layer-5. Finally, the result of RELU-Layer-5 is passed to a batch normalization layer.

Also, before any convolution is applied, each dimensionality reducing block will apply a maximum pooling layer to the input, except for the first dimensionality reducing block, since the input to the first dimensionality reducing block is the original image itself. This is where the "dimensionality reducing" actually occurs, as this layer will reduce the dimension of the input by half. The dimensionality increasing blocks restore the dimension to its original dimension.

The dimensionality increasing blocks are much the same as the dimensionality reducing blocks, except that it applies transposition convolution instead of maximum pooling, and the dimensionality increasing blocks also receive a skip connection from the dimensionality reducing blocks. The specific structure is described below, and also shown in Fig. 4.

First, the dimensionality increasing blocks will apply a transposed convolution (Layer-1) to the input, doubling the dimension of the input; This is where dimensionality increasing occurs. Second, the output of Layer-1 is connected along the channel dimension (Skip-Connection-Input-1) with the Skip Connection from the dimensionality reducing block; This is an example of the structural Skip Connection.

Third, Skip-Connection-Input-1 reduces the dimensions to 32 dimensions through a valid convolution Layer-2 with 32 filters. Fourth, the output of Layer-2 passes again through the same convolutional Layer (Layer-3) with 32 filters (channels). A leaking RELU activation (RELU-Layer-3) is then applied to the output of Layer-3.

Fifth, RELU-Layer-3 is skip-connected with Skip-Connection-Input-1, which makes the output dimensions to 96 (Skip-Connection-Input-2). Sixth, Skip-Connection-Input-2 again passes through a valid convolution Layer (Layer-4) with 32 filters, again reducing the dimensions to 32. Seventh, the output of Layer-4 is again passed through the same Convolutional Layer (Layer-5) with 32 filters (channels). A leaking RELU activation (RELU-Layer-5) is then applied to the output of Layer-5. Finally, the result of RELU-Layer-5 is passed to a batch normalization layer.

After the dimensionality increasing blocks and the dimensionality reducing blocks, the output will be an $N \times N \times 32$ dimensional matrix, where N and N are the dimensions of the image. The $N \times N \times 32$ dimensional matrix will pass through the final valid convolution of 5 filters (channels), and the final output is an $N \times N \times 5$ matrix. Intuitively, this matrix assigns each pixel five values that represent the probability that each pixel should have a label. Among them, 0 is the label of the background, 1 is the label of the sclera, 2 is the label of the iris, 3 is the label of the pupil, and 4 is the label of the caruncula lacrimalis. The final output is calculated by finding the maximum of the 5 values for each pixel and assigning the index of these maximum values to an $N \times N$ matrix, which is the final output mask.

Step S3: Extract the pupil center from the segmented pupil and obtain the pupil center line in the vertical direction.

In the above Step S3, the pupil center is the average X and Y coordinates of all pupil pixels segmented from the first eye position image, and the pupil center line is obtained by drawing a vertical line through the average value of the X coordinates.

Step S4: Determine the distance between junction points of the sclera, the iris or the pupil on the central line of the pupil and the background, and calculating the palpebral fissure height using the distance.

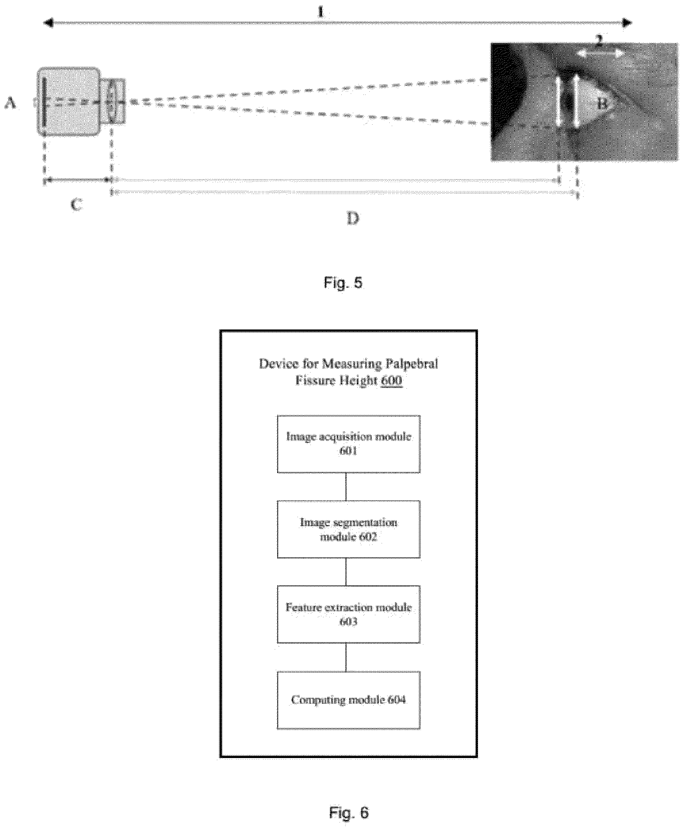

As shown in Fig. 5, the palpebral fissure height is calculated according to the principle of similar triangles in Step S4, and the calculation formula is as follows:
palpebral fissure height B = the pixel distance A of the palpebral fissure height $\times$ a single pixel width and length $\times$ the distance D from the palpebral fissure to the camera lens $\div$ the distance C from camera film to the lens of the camera, wherein the pixel distance (A) of the palpebral fissure height is the distance between the intersection of the sclera, the iris or the pupil and the background on the center line of the pupil.

Further, in Step S4, the distance D from the palpebral fissure to the camera lens = the distance 1 from the canthal latch point to the camera lens - the ocular prominence 2 ( or exophthalmia degree)

Further, ocular prominence 2 is taken as the average value of the normal ocular prominence . From the point of view of statistical averages, the average of normal ocular prominence can be 12-14 mm. In actual operation, the distance 1 from the canthal latch point to the camera lens is known, the distance C from the camera film to the camera lens is also known, the pixel distance A of the palpebral fissure height can be obtained from the first eye position image, and the width and length of a single pixel are also known and fixed values, so the value of the palpebral fissure height can be calculated.

In the process of training the neural network, the definition of loss function is introduced in order to verify the correctness of the final output or result after training the neural network. The loss function is a function that calculates the distance between the current and expected outputs of the algorithm, which provides a numerical indicator of the model's performance. Loss function is an important component of neural networks. A compound loss function is designed in this application, which includes a focal loss, a generalized dice loss, a surface loss and a boundary aware loss.

Firstly, the meaning of the parameters TP, FP, TN and FN in the calculation of loss function should be introduced:

TP: True Positive, which indicates that the predicted result is positive and the actual result is also positive; that is, the prediction is correct;

FP: False Positive, which indicates that the predicted result is positive and the actual result is negative; that is, a positive result is predicted incorrectly and no negative result is predicted;

TN: True Negative, which indicates that the predicted result is negative and the actual result is also negative; that is, the negative result is predicted correctly; and

FN: False Negative, which indicates that the predicted result is negative and the actual result is positive; that is, a negative result is predicted incorrectly, and a positive result is not predicted.

Generalized Dice Loss (GDL) is derived from the $F_1$ score as specified in Formula 2 below, as a supplement to ordinary accuracy. While common accuracy can be a good indicator of a model's performance, it may not be sufficient when it comes to unbalanced data. The commonly used loss function in semantic segmentation schemes is the Intersection over Union (IoU) loss, that is, the ratio of intersection over union between one or more "bounding boxes" and one or more "ground truths." IoU is defined by Formula 1 below. The Dice Factor specified in Formula 3 below can be derived from Formulas 1 and 2, and the Dice Loss can be derived from the dice coefficient(s) in Formula 4 below. Generalized dice loss is just an extension of dice loss. Since the simple dice loss applies to only two split classes, the generalized dice loss is used to deal with multiple split classes; in our case, the five split classes mentioned in the label section. The formulas are as follows:

$$IoU = \frac{Intersection}{Union} = \frac{TP}{FP+TP+FN} \qquad (1)$$

$$F_1 = 2 \times \frac{Precision \times Recall}{Precision + Recall} = \frac{2TP}{2TP+FP+FN} \qquad (2)$$

$$Dice\ Factor = \frac{2Intersection}{Intersection + Union} \qquad (3)$$

$$Dice\ Loss = 1 - Dice\ Factor \qquad (4)$$

wherein *Precision* refers to the precision of classification model indicators, *Recall* refers to the recall rate, *Intersection* refers to the intersection, and *Union* refers to the union.

Focal Loss is a variation of standard cross-entropy loss in that the focal loss attempts to focus on hard negative samples. The focal loss function can be derived from the following aspects: $P_T$ is defined in Formula 5 below, where P stands for a ground truth. With the definition of composite $P_T$, focal loss can be described by Formula 6 below, where $a_T$ and $\gamma$ are hyperparameters. When $\gamma$ is zero, the focal loss collapses into a cross-entropy loss. $\gamma$ is 2 in the example, and $\gamma$ provides a moderating effect that takes losses from easy examples when the ground truth is small. As the model learns the easy examples, the function moves to the harder ones. The formulas involved are as follows:

$$P_T = \begin{cases} P & if\ y = 1 \\ 1 - P & otherwise \end{cases} \qquad (5)$$

$$FL(T_P) = -a_T(1 - P_T)^\gamma log \quad (P_T) \qquad (6)$$

The above y specifies the accuracy class (ground truth) label of the training set for the classification of supervised learning techniques, and p is the estimated probability of the label y = 1.

A semantically defined boundary in a Boundary Aware Loss (BAL) is a separated area based on a class label. The loss of each pixel is weighted according to its distance from the two nearest fragments, introducing edge awareness. In this application, the Canny edge detector in the open cv is used to generate the boundary pixel, which is further enlarged by two pixels to reduce confusion at the boundary. After the value of these boundary pixels is amplified a certain number of times, and then added to a traditional standard cross-entropy loss entropy to increase the model's attention to the boundary.

Surface Loss (SL) is a distance metric based on the image contour space that preserves small, infrequent structures of high semantic value. BAL tries to maximize the probability of correct pixels near the boundary, while GDL provides a stable gradient for unbalanced conditions. In contrast to the BAL AND GDL, SL measures the loss of each pixel based on its distance from the ground-truth boundary of each category, effectively restoring small areas that are overlooked by area-based losses. Surface loss calculates the distance of a single pixel to the boundary of each label group and normalizes this distance against the size of the image. These calculated results are combined with the results predicted by the model, and the mean is obtained.

The final compound loss function is given in Formula 7 as follows:

*Compound loss* = $a_1 \times$ *Surface loss* + $a_2 \times$ *Focal loss* + $a_3 \times$ *Generalized Dice Loss* + $a_4 \times$ Boundary Aware Loss $\times$ *Focal loss*(7)

wherein the variables $a_1$, $a_2$, $a_3$ and $a_4$ are hyperparameters. In the specific circumstances of this application, $a_1$ relates to the number of sessions during the training period; $a_2$ is 1; $a_3$ is 1 - $a_1$; and $a_4$ is 20.

On the one hand, as shown in Fig. 6, this application provides a measuring apparatus for palpebral fissure height, including image acquisition module 601, image segmentation module 602, feature extraction module 603 and calculation module 604, in which:

The image acquisition module 601 is configured to capture the first eye position image in front of eyeballs by photography when the user looking straight ahead in the near-infrared light field of 700-1200 nm;

The image segmentation module 602 adopts the neural network training to segment the background, the iris, the sclera and the pupil from the first eye position image;

The feature extraction module 603 is configured to extract the pupil center from the segmented pupil and obtain the pupil center line in the vertical direction; and

The computing module 604 is configured to calculate the distance between junction points of the sclera, the iris or the pupil on the central line of the pupil and the background, a, and to calculate the palpebral fissure height by using this distance.

On the one hand, the present application provides a computer-readable storage medium in which at least one program code is stored, and the at least one program code is loaded and executed by a processor to perform the present method of measuring the palpebral fissure height.

In the exemplary embodiment, a computer readable storage medium is also provided, including a memory storing at least one program code, which is loaded by a processor and executed to perform the method of measuring the eye crack width in the embodiment. For example, the computer readable storage medium can be a Read-Only Memory (ROM), a Random Access Memory (RAM), a Compact Disc Read-Only Memory (CDROM), a magnetic tape, a floppy disk, optical data storage devices, etc.

A person of ordinary skill in the art may understand that all or part of the steps to implement the above embodiments may be performed by hardware, or by a program in hardware related to at least one program code, which may be stored in a computer readable storage medium, such as read-only memory, disk or optical disc.

The above is only a preferred embodiment of this application and is not intended to limit this application. Any modification, equivalent replacement, improvement, etc. made within the spirit and principles of this application shall be covered by this application.

**Claims**

1. A method for measuring palpebral fissure height, using a camera to obtain an eye image, comprising:

Acquiring a first eye position image in front of eyeballs by photography when a user looking straight ahead in a near-infrared light field of 700-1200 nm;

segmenting a background, an iris, a sclera and a pupil from the first eye position image by a training method of a neural network;

extracting a center of the pupil from the segmented pupil, and obtaining a center line of the pupil in a vertical direction; and

determining a distance between junction points of the sclera, the iris or the pupil on the center line of the pupil and the background, and calculating a palpebral fissure height using the distance.

2. The method according to Claim 1, wherein the pupil center is an average value of X and Y coordinates of all pupil pixels of segmented from the first eye position image, and the pupil center line is obtained by drawing a vertical line through the average value of the X coordinates.

3. The method according to Claim 1, wherein the training method of the neural network segments the background, the iris, the sclera and the pupil from the first eye position image by:

adopting a combination of UNet and DenseNet neural network models as a model for the neural network,

taking the first eye position image as an input to the model for the neural network,

adopting the UNet neural network model to reduce and increase a dimension of the input,

transmitting an output of a dimensionality reducing block to a corresponding dimensionality increasing block by a skip connection, and

each dimensionality reduction block and upsampling each dimensionality increasing block using the DenseNet neural network model.

4. The method according to Claim 3, further comprising verifying a training effect of the neural network by a loss function, wherein the loss function is a compound loss that comprises a focal loss, a generalized dice loss, a surface loss and a boundary aware loss.

5. The method according to Claim 4, wherein the compound loss is calculated as follows:

$$\begin{aligned} \text{compound loss} \\ = a_1 \times \text{ surface loss } + a_2 \times \text{ focal loss} \\ + a_3 \times \text{ generalized dice loss} \\ + a_4 \times \text{ boundary aware loss } \times \text{ focal loss} \end{aligned}$$

wherein $a_1$, $a_2$, $a_3$ and $a_4$ are hyperparameters, $a_1$ is related to a number of duration(s) during training, $a_2 = 1$, $a_3 = 1 - a_1$, and $a_4 = 20$.

6. The method according any of Claims 1-5, wherein the palpebral fissure height is:

palpebral fissure height (B) = a pixel distance (A) of the palpebral fissure height × a single pixel width and length × a distance (D) from the palpebral fissure to a lens of the camera = a distance (C) from a film in the camera to the lens of the camera,

wherein the pixel distance (A) of the palpebral fissure height is a distance between the intersection of the sclera, the iris, or the pupil and the background on the pupil center line.

7. The method according to Claim 6, wherein the distance (D) from the palpebral fissure to the lens of the camera is the distance from a canthal latch point to the lens of the camera (1), minus an ocular prominence (2).

8. The method according to Claim 7, wherein the ocular prominence (2) is an average value of a normal ocular prominence.

9. A apparatus for measuring palpebral fissure height, comprising:

an image acquisition module, configured to acquire a first eye position image in front of eyeballs by photography when a user looking straight ahead in a near-infrared light field of 700-1200nm;

an image segmentation module, configured to segmenting a background, an iris, a sclera and a pupil from the first eye position image by a training method of a neural network;

a feature extraction module, configured to extract a center of the pupil from the segmented pupil, and obtain a center line of the pupil in a vertical direction; and

a computing module, configured to determine a distance between junction point of the sclera, the iris or the pupil on the center line of the pupil and the background, and calculating an palpebral fissure height using the distance.

10. A computer-readable storage medium, configured to have at least one program code that is loaded and executed by a processor to perform the method described in any of Claims 1 to 8.

| S1 |
| Acquiring a first eye position image in front of eyeballs by photography when a user looking straight ahead in a near-infrared light field of 700-1200 nm |

| S2 |
| Segmenting a background, an iris, a sclera and a pupil from the first eye position image by a training method of a neural network training |

| S3 |
| Extracting a center of the pupil from the segmented pupil and obtaining a center line of the pupil in a vertical direction |

| S4 |
| solving for the distance between junction points of the sclera, the iris or the pupil on the central line of the pupil and the background, and calculating the palpebral fissure height using the distance |

Fig. 1

Input — Final Convolution

Dimensionality decreasing block 1 — Skip connection 1 — Dimensionality increasing block 4

Dimensionality decreasing block 2 — Skip connection 2 — Dimensionality increasing block 3

Dimensionality decreasing block 3 — Skip connection 3 — Dimensionality increasing block 2

Dimensionality decreasing block 4 — Skip connection 4 — Dimensionality increasing block 1

Dimensionality decreasing block 5

⊻ Maximum Pooling

○ Valid Convolution

Fig. 2

if not first, max-pooling

input → n×n×α —same conv→ n×n×32 —relu→ n×n×32 ———→ concat ——→ n×n×(32+α)
K= (3, 3)
P= (1, 1)

skip connection

—valid conv→ n×n×32 —same conv→ n×n×32 —relu→ n×n×32 ———→ concat —→n×n×(64+α)
K= (1, 1)        K= (3, 3)
P=0             P= (1, 1)

skip connection

—valid conv→ n×n×32 —same conv→ n×n×32 —relu→n×n×32 —batch normalization→ output
K= (1, 1)        K= (3, 3)
P=0             P= (1, 1)

Fig. 3

skip-connection

input —transposed convolution→ n×n×32 ———→ n×n×64 —valid conv→ n×n×32 —same conv→n×n×32
K= (3, 3)                    K= (1, 1)        K= (3, 3)
P= (1, 1)                   P=0             P= (1, 1)

skip connection

—relu→ n×n×32 ———→ concat ———→n×n×96 —valid conv→ n×n×32 ——→
K= (1, 1)
P=0

—same conv→n×n×32 —relu→ n×n×32 —batch normalization→ output
K= (3, 3)
P= (1, 1)

Fig. 4

Fig. 5

Fig. 6

## INTERNATIONAL SEARCH REPORT

| International application No. |
|---|
| **PCT/CN2023/113527** |

### A. CLASSIFICATION OF SUBJECT MATTER

G06T7/00(2017.01)i; G06T7/11(2017.01)i; G06V10/764(2022.01)n; G06V10/82(2022.01)n; G06N3/0464(2023.01)n

According to International Patent Classification (IPC) or to both national classification and IPC

### B. FIELDS SEARCHED

Minimum documentation searched (classification system followed by classification symbols)

G06T, G06V, G06N, A61B

Documentation searched other than minimum documentation to the extent that such documents are included in the fields searched

Electronic data base consulted during the international search (name of data base and, where practicable, search terms used)

VEN, CNTXT, USTXT, DWPI, CNKI, IEEE: 眼裂, 睑裂, 高度, 宽度, 虹膜, 巩膜, 瞳孔, 中心, 神经网络, 升维, 降维, 跳, 采样, 特征提取, UNet, DenseNet, eye, fissure, fissure, height, width, iris, sclera, pupil, center, neural network, dimension, dimension reduction, jump, sampling, feature extraction

### C. DOCUMENTS CONSIDERED TO BE RELEVANT

| Category* | Citation of document, with indication, where appropriate, of the relevant passages | Relevant to claim No. |
|---|---|---|
| PX | CN 115908237 A (SHANGHAI BAIYI MEDICAL TECHNOLOGY CO., LTD.) 04 April 2023 (2023-04-04) claims 1-10 | 1-10 |
| Y | CN 111938567 A (SHANGHAI JIAO TONG UNIVERSITY) 17 November 2020 (2020-11-17) description, paragraphs [0067]-[0110], and figures 1 and 4 | 1-2, 6-10 |
| Y | CN 210612103 U (SICHUAN ZHONGKE XINGMEI HOSPITAL INVESTMENT CO., LTD.) 26 May 2020 (2020-05-26) description, paragraph [0027], and figures 1-2 | 1, 2, 6-10 |
| Y | US 2021298595 A1 (KAOHSIUNG MEDICAL UNIVERSITY et al.) 30 September 2021 (2021-09-30) description, paragraphs [0006]-[0060], and figures 1-4 | 1, 2, 6-10 |
| Y | CN 104657722 A (WUXI SANGNI'AN SCIENCE & TECHNOLOGY CO., LTD.) 27 May 2015 (2015-05-27) description, paragraphs [0006]-[0010] | 1, 2, 6-10 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 October 2023** | **02 November 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/113527**

| C. | DOCUMENTS CONSIDERED TO BE RELEVANT | |
|---|---|---|
| Category* | Citation of document, with indication, where appropriate, of the relevant passages | Relevant to claim No. |
| Y | CN 110705468 A (SICHUAN UNIVERSITY) 17 January 2020 (2020-01-17) description, paragraphs [0007]-[0016] | 1, 2, 6-10 |
| A | CN 111127484 A (BEIJING XBENTURY NETWORK TECHNOLOGY CO., LTD.) 08 May 2020 (2020-05-08) entire document | 1-10 |

Form PCT/ISA/210 (second sheet) (July 2022)

**INTERNATIONAL SEARCH REPORT**
Information on patent family members

International application No.

**PCT/CN2023/113527**

| Patent document cited in search report | | | Publication date (day/month/year) | Patent family member(s) | | | Publication date (day/month/year) |
|---|---|---|---|---|---|---|---|
| CN | 115908237 | A | 04 April 2023 | None | | | |
| CN | 111938567 | A | 17 November 2020 | None | | | |
| CN | 210612103 | U | 26 May 2020 | None | | | |
| US | 2021298595 | A1 | 30 September 2021 | WO | 2020019286 | A1 | 30 January 2020 |
| | | | | CN | 112384127 | A | 19 February 2021 |
| CN | 104657722 | A | 27 May 2015 | None | | | |
| CN | 110705468 | A | 17 January 2020 | None | | | |
| CN | 111127484 | A | 08 May 2020 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023113527 W
- CN 202210989506
- CN 202010803761
- CN 201910939612X